# FASCICULE DE BREVET EUROPEEN

(11) **EP 1 272 234 B1**
(45) Date de publication et mention de la délivrance du brevet: **01.07.2009**
(21) Numéro de dépôt: 01921442.8
(22) Date de dépôt: 30.03.2001
(51) Int. Cl.: A61L 27/20, A61L 31/04, A61L 33/08

(54) **PROTHESE VASCULAIRE IMPREGNEE DE DEXTRANE RETICULE**
MIT VERNETZTEM DEXTRAN IMPRÄGNIERTE GEFÄSSPROTHESE
VASCULAR PROSTHESIS IMPREGNATED WITH CROSSLINKED DEXTRAN

(30) Priorité: 10.04.2000 FR 0004554
(43) Date de publication de la demande: 08.01.2003
(73) Titulaire: THERAPEUTIQUES SUBSTITUTIVES, F-93430 Villetaneuse (FR)
(72) Inventeur: MACHY, Delphine, F-95610 Eragny sur Oise (FR); JOZEFONVICZ, Jacqueline, F-60260 Lamorlaye (FR); LETOURNEUR, Didier, F-92350 le Plessis Robinson (FR)
(74) Mandataire: Goulard, Sophie
(86) Numéro de dépôt international: PCT/FR2001/000964
(87) Numéro de publication internationale: WO 2001/076652

(56) Documents cités:
- WO-A-00/02596
- US-A- 4 740 594
- US-A- 4 743 258
- US-A- 4 808 709
- US-A- 5 415 619
- US-A- 5 693 625

## Description

La présente invention se rapporte à une prothèse vasculaire imprégnée de dextrane réticulé et/ou de dérivé fonctionnalisé de dextrane réticulé, ainsi qu'à un procédé de préparation d'une telle prothèse.

En chirurgie vasculaire réparatrice, l'utilisation de substituts d'origine synthétique, tels que les prothèses en polyester, en nylon, en polypropylène, en polyacrylonitrile, en polyuréthanne, en polyétheruréthanne, en polyéthylènetéréphtalate ou en polytétrafluoroéthylène expansé, est une alternative à la greffe biologique.

Pour rendre ces substituts synthétiques biocompatibles et, dans le cas où les substituts ne sont pas imperméables en eux-mêmes, pour leur conférer des propriétés d'imperméabilité, leur surface peut être revêtue de substances d'origine biologique, telles que des protéines plasmatiques (albumine ou fibrine) ou des protéines de la matrice extracellulaire (collagène, gélatine ou fibronectine).

Ces protéines présentent néanmoins l'inconvénient de provenir de sources animales ou humaines, d'où un risque potentiel de contamination par des agents pathogènes et des toxines.

Il a déjà été proposé de revêtir la surface de ces substituts synthétiques par de l'alginate, carboxyméthylé ou non (Brevet US 5 415 619). Toutefois, on observe une coagulation rapide du sang à la surface de 1a prothèse et une réponse inflammatoire importante après implantation de ce type de prothèse.

Il a également déjà été proposé, notamment dans le brevet US 4 743 258, un dispositif compatible avec le milieu sanguin, comprenant un matériau de base polymérique et un polymère hydrosoluble substantiellement non-ionique, directement attaché à la surface dudit matériau de base, ce polymère hydrosoluble pouvant être choisi parmi les polymères de type acrylamide ou méthacrylamide ; la polyvinylpyrrolidone ; les alcools polyvinyliques partiellement ou complètement saponifiés ; le polyéthylène glycol et le dextrane non réticulé. Cependant l'utilisation de tels polymères à titre de revêtement de prothèses vasculaires n'est pas satisfaisante dans la mesure où ils se retrouvent rapidement libérés dans la circulation sanguine, du fait de leur solubilité en milieu aqueux entraînant ainsi l'élimination du revêtement de la prothèse.

D'autre part, des prétraitements peuvent être effectués avec le sang du patient, avant implantation de la prothèse. Ces méthodes sont toutefois limitées car elles nécessitent des transfusions du patient, qui prennent du temps et ne peuvent pas être entreprises en cas d'urgence. En outre, elles ne peuvent pas être utilisées dans le cas où les patients sont sous traitement anticoagulant, ce qui est souvent le cas.

La Demanderesse s'est donc donné pour but de pallier les inconvénients de l'art antérieur et de pourvoir à une prothèse vasculaire revêtue d'une substance qui ne soit pas d'origine animale, la prothèse résultante étant à la fois imperméable et souple. En outre, la prothèse munie de son revêtement doit être non toxique et non thrombogène, ne pas déclencher de réaction inflammatoire lorsqu'elle est implantée *in* vivo, tout en conservant son intégrité après implantation.

La présente invention a donc pour objet une prothèse vasculaire souple, caractérisée en ce qu'elle comprend un support synthétique imprégné d'au moins un dextrane réticulé de façon covalente et/ou d'au moins un dérivé fonctionnalisé de dextrane réticulé de façon covalente.

Au sens de la présente invention, on entend par « souple » une prothèse qui est aisément manipulable par le chirurgien lors de l'implantation, et qui est suffisamment flexible pour subir aisément des pliures et des torsions sans que la forme tubulaire de la prothèse ne s'affaisse et sans que les parois ne viennent s'accoler l'une à l'autre.

Le dextrane peut être défini comme un polysaccharide constitué d'un enchaînement d'unités α-D-glucopyranosiques reliées entre elles par des liaisons α(1-6).

Le support synthétique utilisé dans la prothèse selon la présente invention consiste par exemple en du polyester, du nylon, du polypropylène, du polyacrylonitrile, du polyuréthanne, du polyétheruréthanne, du polyéthylènetéréphtalate (tissé ou tricoté) ou du polytétrafluoroéthylène expansé.

La prothèse vasculaire souple selon l'invention présente l'avantage d'être imperméable eu égard à la présence du dextrane réticulé, et biocompatible (c'est-à-dire qu'elle ne déclenche pas une réponse biologique défavorable chez le sujet receveur) du fait de la présence du dérivé fonctionnalisé de dextrane réticulé. Elle est apte à la stérilisation par les rayonnements gamma ou par l'oxyde d'éthylène, traitements qui n'altèrent ni son imperméabilité, ni sa souplesse. Elle présente également l'avantage de conserver des caractéristiques chimiques et physico-chimiques constantes après un contact permanent, pendant 24 heures, de la prothèse dans un flux d'eau en circuit fermé, de façon à reproduire les conditions d'implantation dans la circulation sanguine. Dans ces conditions, la quantité de dextrane libéré atteint un plateau qui représente moins de 1 % en poids seulement du dextrane utilisé pour effectuer le revêtement de la prothèse.

L'utilisation du dextrane et/ou de dérivés fonctionnalisés du dextrane pour enduire le support prothétique écarte tout risque de contamination par des agents pathogènes de source animale. En outre, la prothèse conforme à la présente invention présente l'avantage de ne pas être thrombogène (elle n'active pas le système de la coagulation) et de ne déclencher aucune réaction inflammatoire chez le sujet receveur.

Des dérivés fonctionnalisés du dextrane utilisables pour imprégner la prothèse vasculaire selon la présente invention sont par exemple ceux qui sont décrits dans la Demande internationale PCT publiée sous le numéro WO 99/29734 ou dans l'article de D. LOGEART-AVRAMOGLOU et J. JOZEFONVICZ paru dans J. Biomed. Mater. Res. (Appl. Biomater.) 48, 578-590, 1999.

De tels dérivés, dont la préparation est décrite dans la Demande internationale PCT WO 99/29734, répondent par exemple à la formule générale DMCₐB_{b}Su_{c}S_{d} dans laquelle :
D représente une chaîne polysaccharidique, constituée par des enchaînements d'unités α-D-glucopyranosiques reliées entre elles par des liaisons α(1-6),
MC représente des groupes méthylcarboxylates,
B représente des groupes carboxyméthylbenzylamides,
Su représente des groupes sulfates,
S représente des groupes sulfonates, et
a, b, c et d représentent le degré de substitution (ds), exprimé par rapport au nombre de fonctions hydroxyles libres dans une unité glucosidique du dextrane, respectivement en groupements MC, B, Su et S, a étant égal à 0 ou ≥ à 0,2, b étant égal à 0 ou ≥ à 0,1, c étant égal à 0 ou ≥ à 0,1 et d étant égal à 0 ou ≤ à 0,15, à condition que lorsque d = 0, a et/ou b sont # 0.

Parmi ces dérivés fonctionnalisés du dextrane, on peut plus particulièrement utiliser ceux qui sont sélectionnés dans le groupe constitué par :
- les dextranes fonctionnalisés dans lesquels a ≥ 0,5, c est égal à 0 ou ≤0,5 et d ≤ 0,15 ou égal à 0 et dont la masse molaire moyenne en poids est comprise entre 3 000 et 5 000 000 g/mol,
- les dextranes fonctionnalisés dans lesquels a ≥ 0,3, c est égal à 0 ou ≤ 0,4 et d ≤ 0,15 ou égal à 0 et dont la masse molaire moyenne en poids est comprise entre 3 000 et 5 000 000 g/mol,
- les dextranes fonctionnalisés dans lesquels a ≥ 0,5, c est égal à 0 ou ≤ 0,4 et d ≤ 0,15 ou égal à 0 et dont la masse molaire moyenne en poids est comprise entre 3 000 et 5 000 000 g/mol, et
- les dextranes fonctionnalisés dans lesquels a ≥ 0,2, c ≥ 0,3 et d ≤ 0,15 ou égal à 0 et dont la masse molaire moyenne en poids est comprise entre 3 000 et 5 000 000 g/mol.

A titre d'exemples supplémentaires de dérivés fonctionnalisés du dextrane utilisables pour imprégner la prothèse vasculaire selon la présente invention, on peut également citer :
1) les dérivés de dextrane décrits dans le Brevet européen 0 146 455, qui comportent de manière statistique :
   · au moins 35% environ de motifs B constitués de motifs osides A substitués par des radicaux possédant une fonction carboxyle répondant à la structure -O-(CH₂)ₙ-R-COO- dans laquelle R représente une simple liaison ou un groupe -CO-NH-(CH₂)ₙ·-, n étant un nombre compris entre 1 et 10 et n' étant compris entre 1 et 7,
   · au moins 3% environ de motifs D, c'est-à-dire de motifs osides A substitués par une chaîne comportant un groupe de structure : 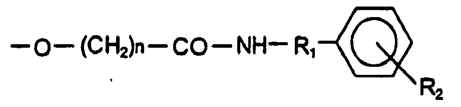
   · dans laquelle n est tel que défini ci-dessus, R₂ représente un anion d'un sel minéral ou organique physiologiquement tolérable, et R₁ représente une simple liaison, un groupe -CH₂- ou un groupe : 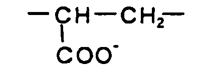
   · éventuellement, des motifs osides A non substitués et/ou des motifs C constitués de motifs A substitués par des radicaux de structure suivante, dans laquelle R₁ et n sont tels que définis ci-dessus : 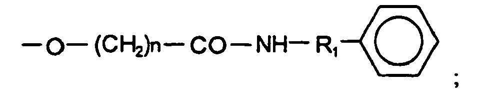
2) les dérivés de dextrane décrits dans le Brevet européen 0 428 182, qui comprennent des motifs A et C et au moins 35% de motifs B, ces motifs étant tels que définis ci-dessus en rapport avec le Brevet européen 0 146 455.

Selon un mode de réalisation avantageux des prothèses vasculaires conformes à la présente invention, le rapport pondéral entre le dérivé fonctionnalisé de dextrane et le dextrane est compris entre 1/99 et 30/70, de préférence compris entre 3/97 et 7/93, de manière encore préférée égal à 5/95 environ*.*

Selon un autre mode de réalisation avantageux des prothèses vasculaires conformes à la présente invention, ledit support synthétique est imprégné, en outre, d'au moins un polysaccharide naturel ou synthétique fonctionnalisé au moins par des fonctions carboxylates et/ou sulfates, réticulé de façon covalente. Un tel polysaccharide fonctionnalisé est par exemple l'héparine.

Le rapport pondéral entre le polysaccharide fonctionnalisé et le dextrane est avantageusement compris entre 1/99 et 30/70, de préférence entre 1/99 et 20/80.

Lorsque la prothèse vasculaire conforme à la présente invention comprend à la fois un dérivé fonctionnalisé de dextrane et un polysaccharide fonctionnalisé, le rapport pondéral entre le dérivé fonctionnalisé de dextrane et le polysaccharide fonctionnalisé est avantageusement compris entre 1/99 et 99/1.

Lorsque des dérivés fonctionnalisés de dextrane et/ou des polysaccharides naturels ou synthétiques fonctionnalisés par au moins des fonctions carboxylates et/ou sulfates, tels que définis ci-dessus, sont présents dans la prothèse conforme à l'invention, ils confèrent à cette dernière des activités biologiques spécifiques.

En particulier, la présence de dérivés fonctionnalisés de dextrane confère à la prothèse conforme à la présente invention la possibilité de développer des interactions spécifiques avec le milieu biologique dans lequel elle est implantée ; on observe notamment une prolifération des cellules endothéliales humaines à l'intérieur de la prothèse, processus qui favorise l'intégration de la greffe dans le milieu biologique.

En outre, en fonction de leurs degrés de substitution en différents groupements fonctionnalisés, les dérivés fonctionnalisés de dextrane et les polysaccharides fonctionnalisés décrits ci-dessus peuvent présenter des propriétés cicatrisantes, des activités anti-complémentaires et de substitut du plasma sanguin, une activité modulatrice de la prolifération cellulaire ou encore des propriétés anticoagulantes.

La présente invention a également pour objet un procédé de préparation d'une prothèse vasculaire telle que décrite ci-avant, caractérisé en ce qu'il comprend les étapes suivantes :
a) préparation d'une solution d'au moins un dextrane et/ou d'au moins un dérivé fonctionnalisé de dextrane,
b) imprégnation du support synthétique à l'aide de cette solution,
c) réticulation dudit dextrane et/ou dudit dérivé fonctionnalisé de dextrane.

La solution préparée lors de l'étape a) peut consister en une solution aqueuse, classiquement à base d'eau osmosée. Elle comprend par exemple entre 1 et 70% (m/V) de dextrane, ou entre 1 et 70% (m/V) de dérivé fonctionnalisé de dextrane, ou entre 1 et 70% (m/V) de dextrane et de dérivé fonctionnalisé de dextrane, en fonction du type de dextrane utilisé et de sa masse molaire moyenne en poids.

Le dextrane utilisé dans le procédé selon l'invention présente de préférence une masse molaire moyenne en poids comprise entre 10 000 et 50 000 000 g/mol.

On peut citer, à titre d'exemples, les dextranes T40, T70 et T500, de masses molaires moyennes en poids respectivement égales à environ 40 000, 70 000 et 460 000 g/mol, commercialisés par la société Pharmacia Biotech, et le dextrane 5M de masse molaire moyenne en poids égale ou supérieure à 5.10⁶ g/mol, commercialisé par la société Sigma. En fonction du type de dextrane utilisé, la solution préparée lors de l'étape a) du procédé selon la présente invention comprend par exemple de 20 à 70% (m/V) de dextrane T40, de 10 à 70% (m/V) de dextrane T70, de 10 à 50% (m/V) de dextrane T500, ou de 1 à 15% (m/V) de dextrane 5M.

Selon un mode de mise en oeuvre avantageux du procédé conforme à la présente invention, ce dernier comprend, entre les étapes a) et b), l'ajout à la solution préparée lors de l'étape a) d'un agent de réticulation, par exemple le STMP (trimétaphosphate de sodium), l'oxychlorure de phosphore (POCl₃), l'épichlorhydrine, les formaldéhydes, les carbodiimides, les glutaraldéhydes ou tout autre composé qui convient pour réticuler un polysaccharide.

Dans ce mode de mise en oeuvre, les étapes b) et c) sont avantageusement effectuées simultanément à un pH compris entre 3 et 10, à une température comprise entre 10 et 40°C et pendant un temps inférieur ou égal à 150 minutes.

Selon un autre mode de mise en oeuvre avantageux du procédé conforme à la présente invention, ce dernier comprend, entre les étapes b) et c), une étape de séchage du support, suivie d'une étape d'imprégnation du support à l'aide d'une solution, dans au moins un solvant organique (tel que l'éther), d'un agent de réticulation.

Un agent de réticulation convenable est par exemple un carbodiimide ou le BDGE (butanediol-1,4 diglycidyl éther), ce dernier pouvant être utilisé à raison de 10 à 30% en moles pour 100 moles de motifs glucosidiques du polysaccharide (dextrane fonctionnalisé ou non), ou tout autre agent de réticulation soluble dans un solvant organique.

Dans ce mode de mise en oeuvre, les étapes b) et c) sont avantageusement effectuées chacune à un pH compris entre 3 et 10 et à une température comprise entre 10 et 40°C l'étape b) étant avantageusement effectuée pendant un temps inférieur ou égal à 150 minutes et l'étape c) pendant un temps compris entre 15 minutes et 18 heures.

Selon une disposition avantageuse de ce mode de réalisation, lesdites étapes successives de préparation d'une solution d'au moins un dextrane et/ou d'au moins un dérivé fonctionnalisé de dextrane, d'imprégnation du support à l'aide de cette solution, de séchage du support, d'imprégnation du support à l'aide d'une solution, dans au moins un solvant organique, d'un agent de réticulation, et de réticulation dudit dextrane et/ou dudit dérivé fonctionnalisé de dextrane sont, après séchage du support, réitérées au moins une fois, de préférence deux à trois fois.

Selon un autre mode de mise en oeuvre avantageux du procédé conforme à la présente invention, l'étape c) de réticulation du dextrane et/ou du dérivé fonctionnalisé de dextrane est suivie d'une étape de lavage de la prothèse, par exemple au moyen d'un mélange d'eau osmosée et d'agents assouplissants et/ou plastifiants tels qu'ils seront décrits ci-après.

Selon un autre mode de mise en oeuvre avantageux du procédé conforme à la présente invention, ladite solution d'au moins un dextrane et/ou d'au moins un dérivé fonctionnalisé de dextrane préparée lors de l'étape a) comprend, en outre, au moins un polysaccharide naturel ou synthétique fonctionnalisé au moins par des fonctions carboxylates et/ou sulfates.

De tels polysaccharides sont tels que décrits précédemment en rapport avec la prothèse vasculaire souple conforme à la présente invention.

Selon encore un autre mode de mise en oeuvre avantageux du procédé conforme à la présente invention, ladite solution d'au moins un dextrane et/ou d'au moins un dérivé fonctionnalisé de dextrane préparée lors de l'étape a) comprend, en outre, au moins un additif choisi parmi les agents plastifiants et les agents assouplissants et/ou un ou plusieurs principes actifs sélectionnés dans le groupe constitué par les agents anticoagulants (tels que l'antivitamine K ou l'aspirine), les agents anti-bactériens et les agents anti-infectieux. Lesdits principes actifs peuvent par exemple être présents dans ladite solution aqueuse à raison de 1 à 10% en poids par rapport aux polysaccharides utilisés.

A titre d'exemples d'agents plastifiants utilisables, on peut citer le glycérol ou le mannitol, présents par exemple à raison de 1 à 10% en volume.

A titre d'exemples d'agents assouplissants utilisables, on peut citer l'acide lactique, l'acide ascorbique, l'éthylène glycol, le propylène glycol ou le sorbitol, présents par exemple à raison de 0,1 à 5% en volume.

Des exemples d'agents anti-bactériens et d'agents anti-infectieux utilisables sont, de façon non limitative, la rifampicine, la minocycline, la chlorhexidine, des ions argent et des compositions à base d'argent.

La présente invention a, en outre, pour objet une prothèse vasculaire souple telle que définie précédemment, caractérisée en ce qu'elle est susceptible d'être obtenue par le procédé selon l'invention tel que défini ci-dessus.

Outre les dispositions qui précèdent, l'invention comprend encore d'autres dispositions qui ressortiront de la description qui va suivre, qui se réfère à des exemples de préparation de prothèses vasculaires selon la présente invention et d'évaluation de leur biocompatibilité cellulaire, ainsi qu'aux dessins annexés, dans lesquels :

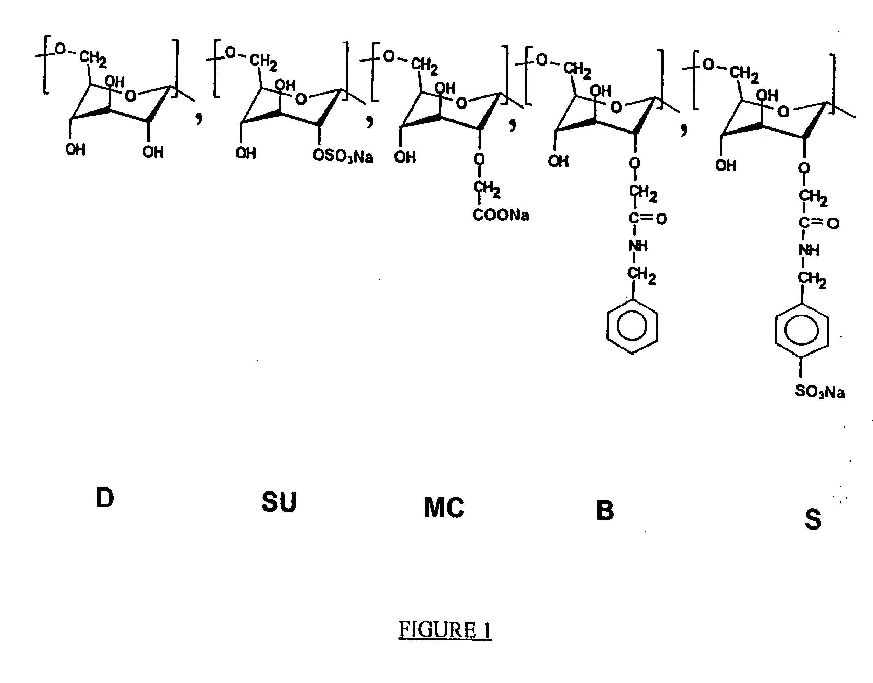

- la figure 1 représente schématiquement la structure d'un dérivé fonctionnalisé de dextrane substitué par les différents groupements chimiques MC, B, Su et S fixés sur les unités glucosidiques D ; à titre d'exemple, la position du substituant sur les différents carbones des unités glucosidiques est présentée sur le carbone 2 ; et

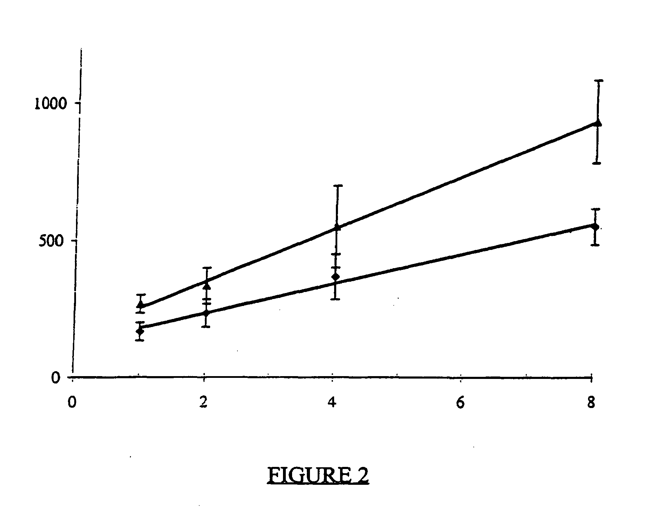

- la figure 2 représente les courbes de croissance des cellules endothéliales humaines permanentes EA.hy 926 sur des échantillons de prothèses vasculaires selon l'invention comprenant soit un dérivé non fonctionnalisé de dextrane (courbes-■-), soit un dérivé non fonctionnalisé de dextrane et un dérivé fonctionnalisé de dextrane (courbe -π-), le temps (en jours) figurant en abscisses et le nombre de cellules par cm² (x 10) en ordonnées.

Il doit être bien entendu, toutefois, que ces exemples sont donnés uniquement à titre d'illustration de l'objet de l'invention, dont ils ne constituent en aucune manière une limitation.

### EXEMPLE 1 : Préparation de prothèses vasculaires selon la présente invention, qui comprennent un support synthétique imprégné d'un dextrane réticulé à l'aide de BDGE.

### 1) Support synthétique, dextrane et agent de réticulation utilisés.

Le support synthétique utilisé consiste en du polyéthylène-téréphtalate (PET) tricoté, fourni par la société Cardial & Bard sous la dénomination commerciale « Dialine I ». Sa perméabilité à l'eau est de 1010 ml/min/cm² selon la norme ISO/DIS 7198:1996. Il se présente sous la forme d'un textile cosselé de 40 à 60 cm de longueur (sous forme étirée) et de 8 à 10 mm de diamètre. Des échantillons de 20 cm de longueur (étirée) sont utilisés dans le cadre du présent exemple.

Le dextrane utilisé est du dextrane 5M de masse molaire moyenne en poids égale à 5.10⁶ g/mol, commercialisé par la société Sigma.

Quant à l'agent de réticulation du dextrane, il s'agit du BDGE (butanediol-1,4 diglycidyl éther, ALDRICH).

### 2) Protocole.

Le protocole de préparation des prothèses vasculaires consiste en des séries de cycles successifs d'imprégnation du support synthétique de PET par une solution de dextrane et de réticulation dans un solvant organique, séparées par des étapes de séchage.

Les solutions de dextrane préparée en vue des étapes d'imprégnation sont des solutions à 9, 9,5 et 10% (m/V) de dextrane 5M dans de l'eau osmosée. A ces solutions sont ajoutés de l'acide lactique (0,7% en volume) et du glycérol (2% en volume). Le pH est ajusté à 7 à l'aide d'une solution de soude 2M.

L'imprégnation du support synthétique en PET est réalisée à l'aide d'une pompe péristaltique qui aspire la solution de dextrane et l'entraîne à l'intérieur de l'échantillon tubulaire, par ses deux extrémités. Le temps d'imprégnation est d'environ 10 secondes à 5 minutes.

L'imprégnation est suivie du séchage des échantillons dans une étuve à 40°C pendant 24 heures.

On effectue ensuite une étape de réticulation du dextrane : les échantillons sont placés dans une solution d'éther, à laquelle on ajoute le BDGE (22,3 moles pour 100 moles de motifs glucosidiques du dextrane). La réticulation est effectuée pendant 18 heures.

Les échantillons sont ensuite séchés pendant une heure sous une hotte aspirante.

On effectue trois cycles successifs d'imprégnation et de réticulation, puis les échantillons sont lavés trois fois trois heures à l'aide d'une solution d'eau osmosée comprenant 0,7% (V /V) d'acide lactique et 2% de glycérol (V/V), de pH 7.

### 3) Résultats.

Pour chaque échantillon, on mesure le taux de revêtement de l'échantillon R (%), qui correspond à l'augmentation de la masse de l'échantillon après son traitement par la solution polysaccharidique (R = (P₂ - P₁ ) / P₁ x 100, P₁ représentant la masse initiale de l'échantillon et P₂ sa masse finale après traitement).

On mesure en outre la perméabilité statique des échantillons selon la norme ISO/DIS 7198:1996, qui consiste à mesurer le débit d'eau passant à travers une surface donnée d'une prothèse échantillonnée, par gravimétrie et sous pression hydrostatique. L'échantillon peut être immergé dans de l'eau pure à température ambiante afin de l'imprégner d'eau avant de procéder à l'essai (« perméabilité Hyd » : perméabilité à l'état hydraté), ou être soumis directement à l'essai (« perméabilité non Hyd » : perméabilité à l'état non hydraté). La perméabilité est le quotient du débit d'eau (en ml/min) par unité d'aire (en cm²): elle est exprimée en ml/min/cm². Dans les exemples qui suivent, sauf mention contraire, les perméabilités mesurées sont des perméabilités statiques obtenues selon la norme ISO/DIS 7198:1996.

Les résultats obtenus pour les prothèses 1 à 3 sont rassemblés dans le tableau I.

**Tableau I**

| Prothèse n° | Concentration de la solution d'imprégnation en dextrane (m/V) | R (%) | Perméabilité Non Hyd | Perméabilité Hyd |
|---|---|---|---|---|
| **1** | 9% | 24 ± 2 | 54 | 1,5 |
| **2** | 9,5% | 22 ± 3 | 50 | 1 |
| **3** | 10% | 27 ± 1 | 14 | 1 |

Les prothèses 1 à 3 présentent le caractère de souplesse requis et des taux de revêtement permettant d'obtenir une bonne étanchéité tout en conservant la souplesse de l'échantillon.

### EXEMPLE 2 : Autre exemple de préparation de prothèses vasculaires selon la présente invention, qui comprennent un support synthétique imprégné d'un dextrane réticulé à l'aide de BDGE.

Le support synthétique, le dextrane et l'agent de réticulation utilisés sont tels que décrits dans l'exemple 1. A la différence de l'exemple 1, le protocole suivi ne comprend que deux cycles successifs d'imprégnation et de réticulation, avec trois lavages de trois heures en fin de protocole. Les solutions d'imprégnation sont constituées de dextrane 5M à la concentration de 10% (m/V), sans glycérol ni acide lactique lors du premier cycle et avec 2% en volume de glycérol pour le deuxième cycle. La concentration en BDGE, identique pour les deux cycles, est de 15, 18 ou 20% (en nombre de moles par rapport à 100 moles de motifs glucosidiques du dextrane) selon les échantillons.

Les résultats obtenus pour les prothèses 4 à 6, qui présentent le caractère de souplesse requis, sont rassemblés dans le tableau II.

**Tableau II**

| Prothèse n° | Quantité de BDGE (% molaire) | R (%) | Perméabilité Non Hyd | Perméabilité Hyd |
|---|---|---|---|---|
| **4** | 20% | 27±1 | 2 | 1 |
| **5** | 18% | 26 ± 0.5 | 14 | 5 |
| **6** | 15% | 25,5 ± 1 | 20 | 6 |

### EXEMPLE 3 : Préparation de prothèses vasculaires selon la présente invention, qui comprennent un support synthétique imprégné d'un dextrane réticulé à l'aide de STMP, ainsi qu'éventuellement un polysaccharide fonctionnalisé.

### 1) Support synthétique, dextranes et agent de réticulation utilisés.

Le support synthétique (PET tricoté) et le dextrane (dextrane 5M) utilisés sont identiques à ceux décrits dans l'exemple 1. L'agent de réticulation consiste en du STMP (trimétaphosphate de sodium, commercialisé par SIGMA).

Le dérivé fonctionnalisé de dextrane utilisé, de masse molaire moyenne en poids est d'environ 106 900 g/mol, est le DMC₃B₂; il répond à la formule générale DMCₐB_{b}Su_{c}S_{d} telle que précédemment décrite, dans laquelle le degré de substitution en groupements méthylcarboxylates (indice a) est égal à 0,87 et le degré de substitution en groupements carboxyméthylbenzylamides (indice b) est égal à 0,26, les indices c et d étant égaux à 0. Son protocole de préparation est tel que décrit dans le Brevet européen publié sous le numéro 0 146 455.

### 2) Protocole.

Une solution de dextrane 5M à 10% (m/V) est préparée dans de la soude 0,2 M en présence de 0,7% (en volume) d'acide lactique et de 2% (en volume) de glycérol. Cette solution peut également comprendre, si nécessaire, le dérivé fonctionnalisé de dextrane DMC₃B₂ auquel cas, le dextrane 5M est mélangé, en phase solide, au dextrane DMC₃B₂ avant d'être mis en solution, la proportion de DMC₃B₂ étant égale à 3% en poids par rapport au poids du dextrane 5M, soit un rapport pondéral de 3/97 entre le dérivé fonctionnalisé de dextrane et le dextrane 5M.

En variante, la solution de dextrane peut également comprendre de l'héparine (agent anticoagulant) à raison de 1%, de 3%, de 5% ou de 10% en poids par rapport au poids du dextrane 5M, soit des rapports pondéraux entre l'héparine et le dextrane respectivement égaux à 1/99, 3/97, 5/95 et 10/90.

La solution d'imprégnation ainsi préparée est chauffée à 40°C pendant 20 minutes, puis le STMP (8 moles pour 100 moles de motifs glucosidiques du dextrane 5M et du dextrane DMC₃B₂, si ce dernier est présent), préalablement dilué dans 0,5 ml de soude 0,2 M, est ajouté à la solution d'imprégnation.

Le support synthétique est imprégné par cette solution de la même façon que décrit dans l'exemple 1, à température ambiante et pendant 10 secondes à 5 minutes. Si le dextrane DMC₃B₂ est présent, ce dernier sera « co-réticulé » avec le dextrane 5M.

La prothèse est séchée pendant 2 heures à 40°C, puis lavée trois fois, pendant 3 heures, à l'aide d'une solution d'eau osmosée comprenant 0,7% (V/V) d'acide lactique et 2% de glycérol (V/V), de pH 7.

### 3) Résultats.

Les résultats obtenus pour les prothèses 7 à 12 sont rassemblés dans le tableau III: les paramètres de quantité de revêtement R et de perméabilités étant mesurés comme indiqué dans l'exemple 1.

**Tableau III**

| Prothèse n° | Composition du revêtement | Perméabilité Non Hyd | Perméabilité Hyd |
|---|---|---|---|
| **7** | dextrane 5M | 0 | 0 |
| **8** | dextrane 5M + DMC₃B₂ | 0 | 0 |
| **9** | Dextrane 5M + héparine (1%) | 3,3 | 0 |
| **10** | dextrane 5M + héparine (3%) | 10 | 0 |
| **11** | Dextrane 5M + héparine (5%) | 30 | 0 |
| **12** | Dextrane 5M + héparine (10%) | 40 | 0 |

Les prothèses 7 à 12 sont souples. La présence du dérivé fonctionnalisé de dextrane DMC₃B₂ ou de l'héparine ne perturbe pas la réaction de réticulation, les perméabilités à l'état hydraté étant identiques dans les échantillons 7 à 12.

### EXEMPLE 4 : Autre exemple de préparation de prothèses vasculaires selon la présente invention, qui comprennent un support synthétique imprégné d'un dextrane réticulé à l'aide de STMP.

Le support synthétique (PET tricoté) et l'agent de réticulation (STMP) sont identiques à ceux utilisés dans l'exemple 3. Le dextrane utilisé est le T500, de masse molaire moyenne en poids égale à 460 000 g/mol, commercialisé par la société Pharmacia Biotech.

On procède de la même façon que dans l'exemple 3, la solution d'imprégnation comprenant 22% (m/V) de dextrane T500, 0,7% (en volume) d'acide lactique et 2% (en volume) de glycérol. Le STMP est présent à raison de 8% (en moles pour 100 moles de motifs glucosidiques du dextrane).

On obtient l'échantillon de prothèse 13, qui présente les propriétés de souplesse et d'imperméabilité requises et dont les caractéristiques sont rassemblées dans le tableau IV :

**Tableau IV**

| Prothèse n° | Composition du revêtement. | R (%) | Perméabilité Non Hyd | Perméabilité Hyd |
|---|---|---|---|---|
| **13** | dextrane T500 | 27 ± 1 | 1,5 | 0,5 |

### EXEMPLE 5 : Autres exemples de préparation de prothèses vasculaires selon la présente invention, qui comprennent un support synthétique imprégné d'un dextrane réticulé à l'aide de STMP.

Le support synthétique (PET tricoté), l'agent de réticulation (STMP) et les dextranes (T500 ou 5M) utilisés sont identiques à ceux décrits dans les exemples 3 et 4.

On procède de la même façon que dans l'exemple 3, la solution d'imprégnation comprenant soit 20% (m/V) de dextrane T500, soit 10% (m/V) de dextrane 5M, ainsi que 0,7% (en volume) d'acide lactique et 2% (en volume) de glycérol. Le STMP est présent à raison de 8% (en moles pour 100 moles de motifs glucosidiques du dextrane).

On fait varier le temps de contact, à savoir l'intervalle de temps séparant l'ajout du STMP à la solution de dextrane et l'imprégnation de l'échantillon à l'aide de cette solution.

On obtient les échantillons de prothèses 14 à 19, qui présentent les propriétés de souplesse et d'imperméabilité requises, et dont les caractéristiques sont rassemblées dans le tableau V :

**Tableau V**

| Prothèse n° | Composition du revêtement | Temps de contact (minutes) | R (%) | Perméabilité Non Hyd | Perméabilité Hyd |
|---|---|---|---|---|---|
| **14** | dextrane T500 | 2 | 31 ± 1,0 | 1 | 0 |
| **15** | dextrane T500 | 15 | 33±1,5 | 0 | 0 |
| **16** | dextrane T500 | 30 | 43 ± 1,0 | 0 | 0 |
| **17** | dextrane 5M | 15 | 24±1,5 | 0,2 | 0 |
| **18** | dextrane 5M | 30 | 25 ± 0,5 | 0 | 0 |
| **19** | dextrane 5M | 45 | 25 ± 0,2 | 0 | 0 |

Des prothèses vasculaires souples et imperméables selon la présente invention peuvent donc être obtenues en variant le temps de contact de l'agent de réticulation avec la solution de dextrane, avant imprégnation du support synthétique.

La réaction de réticulation du dextrane débute dès que l'agent de réticulation est mis en contact avec la solution de dextrane, provoquant une augmentation de la viscosité de la solution d'imprégnation. L'imprégnation du support prothétique doit être réalisée à un stade auquel la solution d'imprégnation n'est ni trop liquide (ce qui donnerait un revêtement trop fin), ni trop visqueuse (l'imprégnation du support prothétique étant alors difficile). Les temps de contact exemplifiés dans le tableau V ci-dessus conviennent pour l'obtention de prothèses aux caractéristiques souhaitées.

### EXEMPLE 6: Stérilisation d'une prothèse selon la présente invention par rayonnements gamma.

Des échantillons de la prothèse 7 préparée dans l'exemple 3 ont été stérilisés par rayonnements gamma, à une dose de 25 kGray. Les perméabilités des prothèses stérilisées ont été mesurées, ainsi que celles de prothèses identiques non stérilisées. Les résultats sont présentés dans le tableau VI.

**Tableau VI**

| Type de revêtement | R (%) | Perméabilité Non Hyd | Perméabilité Hyd |
|---|---|---|---|
| Prothèse non stérile | 25 | 0 | 0 |
| Prothèse stérile | 25 | 0 | 0 |

Les prothèses restent étanches et souples après stérilisation aux rayons gamma. Les rayonnements gamma ne semblent pas dégrader l'aspect des revêtements des prothèses. Au contraire, ils ont probablement un effet analogue à celui d'un agent réticulant : ils créent des radicaux libres capables d'interagir entre eux et qui peuvent former des liaisons covalentes.

### EXEMPLE 7: Mesure de la perméabilité intégrale à l'eau des prothèses vasculaires selon la présente invention.

### 1) Nature des échantillons de prothèses.

On utilise la prothèse 7 préparée dans l'exemple 3, qui comprend 10% (m/V) de dextrane 5M, ainsi qu'une prothèse 20, préparée conformément à l'exemple 3 en utilisant 10% (m/V) de dextrane 5M et 7% (exprimé en poids par rapport à la quantité de dextrane 5M) de DMC₃B₂, soit un rapport pondéral entre le dérivé fonctionnalisé de dextrane et le dextrane 5M égal à 7/93. L'agent de réticulation est le STMP, présent dans la solution d'imprégnation à raison de 8% en moles pour 100 moles de motifs glucosidiques du dextrane.

### 2) Paramètres mesurés.

Pour les prothèses 7 et 20, on mesure :
- la perméabilité statique à l'état hydraté ou non hydraté (mesurée selon la norme ISO/DIS 7198:1996 telle que présentée dans l'exemple 1),
- la perméabilité intégrale à l'eau, évaluée selon la procédure normalisée ISO 7198. Dans le tableau VII ci-dessous figurent la longueur de l'échantillon (1, en cm), le volume d'eau récupéré (V, en ml/min) et la perméabilité intégrale (P, en ml/min/cm²),
- le taux de revêtement R, mesuré comme indiqué dans l'exemple 1,
- le pourcentage d'humidité relative HR, ce paramètre ayant une influence sur la souplesse de l'échantillon. On effectue une pesée de l'échantillon à l'état sec (après passage dans une étuve à 40°C) et à l'état humidifié (échantillon placé dans une cloche à 80% d'humidité), la différence entre ces pesées, rapportée au poids de l'échantillon sec, permettant d'obtenir le pourcentage d'humidité relative HR.

### 3) Résultats.

Les caractéristiques des échantillons 7 et 20, qui présentent notamment les propriétés de souplesse et de perméabilité requises, sont rassemblées dans le tableau VII.

**Tableau VII**

| Prothèse | Perméabilité | Perméabilité | Perméabilité intégrale | | | R (%) | HR (%) |
|---|---|---|---|---|---|---|---|
| n° | Non Hyd | Hyd | 1 | V | P | | |
| 7 | 0 | 0 | 8,4 | 2,1 | 0,08 | 36 | 52 |
| 20 | 0,67 | 0 | 7,3 | 12 | 0,52 | 31 | 45 |

### EXEMPLE 8 : Biocompatibilité cellulaire in vitro des agents de revêtement des prothèses vasculaires selon la présente invention.

Cet exemple a pour but d'étudier la cytocompatibilité des gels de dextrane utilisés comme agent de revêtement des prothèses vasculaires conformes à l'invention.

### I) Préparation des gels de dextrane.

Les gels de dextrane sont préparés de la façon suivante :
- gel de dextrane non fonctionnalisé (gel A) : on prépare une solution à 10 % de dextrane 5M de masse molaire moyenne en poids égale à 5.10⁶ g/mol, commercialisé par la société Sigma dans une solution aqueuse stérile de NaOH 0,2 M contenant 0,7 % d'acide lactique et 2 % de glycérol (solution A);
- gels de dextrane non fonctionnalisé et de dextrane fonctionnalisé (gels **B** à **D) :** on prépare des solutions à 10 % de dextrane 5M tel que décrit ci-dessus et contenant 3 % (p/v) de dextrane fonctionnalisé de formule DMC₂B_{b}Su_{c}S_{d} par rapport au poids de dextrane non fonctionnalisé, dans une solution aqueuse stérile de NaOH 0,2 M contenant 0,7 % d'acide lactique et 2% de glycérol (Solutions B à D). Les indices de substitution a, b, c et d des dextranes de formule DMC₃B_{b}Su_{c}S_{d} utilisés figurent dans le tableau VIII ci-après :

**Tableau VIII**

| **Solutions** | **Dextrane fonctionnalisé utilisé** | | | | **Masse molaire en g/mol** |
|---|---|---|---|---|---|
| | **a** | **b** | **c** | **d** | |
| **B** | 0,81 | 0,20 | 0,13 | 0 | 67000 |
| **C** | 0,61 | 0,39 | 0 | 0 | 63000 |
| **D** | 0,61 | 0,39 | 0,04 | 0 | 63000 |

Les solutions de dextrane A à D ainsi préparées sont chauffées à 40°C pendant 20 minutes, puis l'agent de réticulation (8 moles de STMP pour 100 moles de motifs glucosidiques, tel que décrit ci-dessus à l'exemple 3), préalablement dilué dans 0,5 ml de soude 0,2 M, est ajouté à ces solutions de dextrane.

Les mélanges sont ensuite coulés dans des plaques de culture 12 puits à raison de 150 µl par puits. Lorsque la réaction de réticulation est terminée, les gels ainsi obtenus sont lavés trois fois au PBS stérile.

### II) Etude de l'adhérence des cellules endothéliales EA.hv 926 sur les sets A à D

Des cellules endothéliales humaines EA.hy 926 sont ensemencées à raison de 2.10⁶ cellules dans 3 ml de milieu de culture DMEM contenant 10 % de sérum de veau foetal (SVF) et 2 µCi/ml de proline tritiée (Amersham). Après 24 heures de culture à 37°C, les cellules sont détachées à l'aide d'une solution de trypsine, centrifugées à 1200 tours par minute, puis resuspendues dans un milieu de culture DMEM contenant 10 % de SVF. Les cellules sont ensuite comptées à la cellule de Malassez, diluées et ensemencées sur les plaques contenant les gels de dextrane A à D, à raison de 50 000 cellules par puits. Les plaques de culture sont ensuite mises à incuber à 37°C pendant 3 heures.

A la fin de la période d'incubation, les puits ensemencés sont rincés deux fois au PBS afin d'éliminer les cellules n'ayant pas adhéré sur les gels de dextrane.

Les cellules adhérentes sont ensuite détachées à l'aide d'une solution de trypsine puis les cellules de chaque puits sont mises dans un flacon de comptage contenant 5 ml de liquide scintillant (Optiphase® "Hisafe", Wallack).

La mesure de la radioactivité présente dans chaque flacon est effectuée à l'aide d'un compteur à scintillation (LKB) à raison de 4 comptages d'une minute par flacon.

Le pourcentage d'adhérence des cellules sur les gels B à D est déterminé par rapport à la radioactivité émise dans les puits témoins, à savoir les puits contenant le gel A de dextrane non fonctionnalisé.

Les résultats obtenus figurent dans le tableau IX ci-après :

**Tableau IX**

| **Gels** | **% d'adhérence des cellules par rapport à l'adhérence observée sur le gel A** |
|---|---|
| **A** | **100** |
| **B** | 165 ±19.7 |
| **C** | 184 ± 15,7 |
| **D** | 195,7 ± 6,3 |

Ces résultats montrent que la présence d'un dérivé fonctionnalisé de dextrane permet de favoriser l'adhérence des cellules endothéliales.

Des résultats similaires ont été obtenus en réalisant la même expérience sur des cellules musculaires lisses de rat (CMI).

### III) Etude de la prolifération des cellules endothéliales EA.hv 926 sur les gels A et C

Des cellules endothéliales humaines EA.hy 926 sont ensemencées à raison de 10 000 cellules par cm² dans du milieu de culture DMEM contenant 10 % de SVF, sur les gels de dextrane A et C.

Après 5 jours de culture, les cellules sont rincées trois fois au PBS puis sont détachées avec 500 µl d'une solution de trypsine et mises en suspension dans une solution électrolyte d'Isotron. Les cellules sont ensuite comptées à l'aide d'un appareil de comptage Coulter Counter ®.

Les résultats de prolifération cellulaire observée sur le gel C sont exprimés en pourcentage de la prolifération cellulaire observée sur le gel A et figurent dans le tableau X ci-après :

**Tableau X**

| **Gel** | **% de prolifération des cellules par rapport à la prolifération observée sur le gel A** |
|---|---|
| **A** | 100 |
| **C** | 116,5 ± 0,43 |

Ces résultats montrent que la présence d'un dérivé fonctionnalisé de dextrane permet de favoriser la prolifération des cellules endothéliales.

Des résultats similaires ont été obtenus en réalisant la même expérience sur des cellules musculaires lisses de rat (CMI).

L'ensemble de ces résultats montre que la présence d'un dérivé fonctionnalisé de dextrane selon la présente invention favorise l'adhérence et la prolifération des cellules endothéliales, propriété d'importance pour favoriser l'intégration dans le milieu biologique de la prothèse vasculaire qu'il revêt.

### EXEMPLE 9 : Biocompatibilité cellulaire in vitro des prothèses vasculaires selon la présente invention.

### 1) Protocole.

Pour analyser la cytocompatibilité des prothèses 7 et 8 synthétisées dans l'exemple 3 avec les cellules endothéliales humaines EA.hy 926, des échantillons de 1 cm² sont découpés sur les prothèses, stérilisés par rayonnements gamma et introduits dans des plaques de culture de 24 puits. Des inserts de verre stériles sont placés sur les échantillons afin de les empêcher de s'enrouler sur eux-mêmes une fois immergés dans le milieu de culture.

Les échantillons de prothèses sont maintenus pendant 24 heures dans le milieu de culture, à savoir un milieu DMEM sans pyruvate de sodium comprenant 4500 mg/ml de glucose (GIBCO), supplémenté en L-glutamine (2 mM), en HAT (2% V/V) et en sérum de veau foetal à 10% (GIBCO), puis le milieu est aspiré et les échantillons sont ensemencés avec les cellules endothéliales humaines EA.hy 926 à la densité de 5.10⁴ cellules/cm², chaque échantillon étant réalisé en triple exemplaire. Les échantillons ensemencés sont placés dans un incubateur à 37°C, sous une atmosphère humide contenant 5% de CO₂, dans des conditions statiques. Le milieu est renouvelé tous les deux jours.

La prolifération cellulaire est observée chaque jour pendant 8 jours, au moyen d'un microscope optique inverse. A cette fin, les échantillons sont lavés trois fois dans du PBS (non stérile), fixés dans du formaldéhyde à 4%, lavés de nouveau trois fois dans du PBS, puis colorés au bleu de Coomassie (5%) et enfin décolorés à l'éthanol à 70%, de façon à ce que seules les membranes des cellules restent colorées. Au microscope, les cellules apparaissent en bleu sur fond non coloré.

### 2) Résultats.

La figure 2 représente les courbes de croissance des cellules endothéliales humaines permanentes EA.hy 926 sur les échantillons de prothèses vasculaires 7 (courbe -■-) et 8 (courbe -π-), le temps (en jours) figurant en abscisses et le nombre de cellules par cm² (x 10) en ordonnées.

On constate une différence significative entre la croissance des cellules sur les échantillons 7 et 8 : lorsque le DMC₃B₂ est présent dans le revêtement de la prothèse, le nombre de cellules est plus important au fur et à mesure des jours écoulés, indiquant que la présence du dérivé fonctionnalisé de dextrane dans la prothèse améliore la prolifération des cellules endothéliales humaines EA.hy 926.

Des résultats similaires ont été obtenus avec d'autres cultures de cellules endothéliales, à savoir les cellules endothéliales de veine de cordons ombilicaux humains et les cellules endothéliales d'aorte bovine. Il apparaît donc que, de manière générale, la présence d'un dérivé fonctionnalisé de dextrane dans la prothèse vasculaire selon la présente invention favorise la prolifération des cellules endothéliales, propriété d'importance pour favoriser l'intégration de la greffe dans le milieu biologique.

### EXEMPLE 11 : Etude de la stabilité d'une prothèse vasculaire conforme à l'invention comparée à celle d'une prothèse vasculaire revêtue par du dextrane non réticulé

### 1) Préparation des prothèses vasculaires

Une prothèse vasculaire conforme à l'invention a été préparée en suivant le protocole décrit à l'exemple 3 et en utilisant le support et le dextrane décrits ci-dessus à l'exemple 1 et l'agent de réticulation décrit à l'exemple 3 ci-dessus.

A titre comparatif, une prothèse vasculaire ne faisant pas partie de l'invention a été préparée dans les mêmes conditions, sauf que l'agent de réticulation n'a pas été ajouté à la solution de dextrane.

### 2) Etude de la stabilité du revêtement

Le montage utilisé pour cette étude est constitué d'un bain Marie thermostaté à 37°C et d'une pompe permettant de faire circuler un flux continu d'une solution à l'intérieur d'une prothèse vasculaire. Le débit de la pompe a été réglé à 7.10⁻⁵ m³ par seconde de façon à reproduire les conditions du débit physiologique de la circulation artérielle.

Des prélèvements de la solution de circulation à intervalles de temps réguliers sont effectués pour quantifier la quantité de dextrane libéré. Le dosage du dextrane libéré est effectué de manière indirecte par dosage colorimétrique des glucides en milieu acide sulfurique concentré/phénol selon la méthode de Dubois et al. (Analytical Chemistry, 1956. 3 (28), 350-356), éventuellement après lyophilisation et dilution de la solution de circulation lorsque que les quantités de dextrane libérées sont extrêmement faibles.

Les résultats obtenus pour la prothèse conforme à l'invention (imprégnée de dextrane réticulé) figurent dans le tableau XI ci-après :

**Tableau XI**

| Temps en heures | Quantité de dextrane libéré en µg (prothèse conforme à l'invention) |
|---|---|
| 1 | 25 |
| 2 | 35 |
| 12 | 65 |
| 24 | 70 |

Il n'a pas été possible de mesurer la quantité de dextrane libéré au cours du temps par la prothèse non conforme à l'invention (imprégnée par un dextrane non réticulé) dans la mesure où le dextrane a été libéré en totalité au cours de premier lavage de la prothèse, c'est-à-dire avant même la mise en contact de ladite prothèse avec le flux d'eau en circuit fermé. Cette libération de dextrane a pour conséquence l'effondrement des propriétés d'étanchéité de la prothèse.

En revanche, au bout de 24 heures de circulation, la quantité de dextrane libéré par la prothèse vasculaire conforme à l'invention, c'est à dire imprégnée par un dextrane réticulé de façon covalente, atteint un plateau à 70 µg représentant environ 0,014 % de la quantité totale de dextrane imprégnant la prothèse. Cette quantité négligeable est significative de la très grande stabilité chimique des prothèses vasculaires conformes à l'invention au cours du temps.

## Revendications

1. Prothèse vasculaire souple, **caractérisée en ce qu'**elle comprend un support synthétique imprégné d'au moins un dérivé fonctionnalisé de dextrane réticulé de façon covalente, et éventuellement d'au moins un dextrane réticulé de façon covalente.

2. Prothèse selon la revendication 1, **caractérisée en ce que** le rapport pondéral entre le dérivé fonctionnalisé de dextrane et le dextrane est compris entre 1/99 et 30/70, de préférence compris entre 3/97 et 7/93, de manière encore préférée égal à 5/95 environ.

3. Prothèse selon la revendication 1 ou la revendication 2, **caractérisée en ce que** ledit support synthétique est imprégné, en outre, d'au moins un polysaccharide naturel ou synthétique fonctionnalisé au moins par des fonctions carboxylates et/ou sulfates, réticulé de façon covalente.

4. Prothèse selon la revendication 3, **caractérisée en ce que** le rapport pondéral entre le polysaccharide fonctionnalisé et le dextrane est avantageusement compris entre 1/99 et 30/70, de préférence entre 1/99 et 20/80.

5. Prothèse selon la revendication 3 ou la revendication 4, **caractérisée en ce que** le rapport pondéral entre le dérivé fonctionnalisé de dextrane et le polysaccharide fonctionnalisé est avantageusement compris entre 1/99 et 99/1.

6. Procédé de préparation d'une prothèse vasculaire selon l'une quelconque des revendications précédentes, **caractérisé en ce qu'**il comprend les étapes suivantes :
a) préparation d'une solution d'au moins un dérivé fonctionnalisé de dextrane, et éventuellement d'au moins un dextrane,
b) imprégnation du support synthétique à l'aide de cette solution,
c) réticulation dudit dérivé fonctionnalisé de dextrane, et éventuellement dudit dextrane,

7. Procédé selon la revendication 6, **caractérisé en ce qu'**il comprend, entre les étapes a) et b), l'ajout d'un agent de réticulation à la solution préparée lors de l'étape a).

8. Procédé selon la revendication 7, **caractérisé en ce que** les étapes b) et c) sont effectuées simultanément à un pH compris entre 3 et 10, à une température comprise entre 10 et 40°C et pendant un temps inférieur ou égal à 150 minutes.

9. Procédé selon la revendication 6, **caractérisé en ce qu'**il comprend, entre les étapes b) et c), une étape de séchage du support, suivie d'une étape d'imprégnation du support à l'aide d'une solution, dans au moins un solvant organique, d'un agent de réticulation.

10. Procédé selon la revendication 9, **caractérisé en ce** les étapes b) et c) sont effectuées chacune à un pH compris entre 3 et 10 et à une température comprise entre 10 et 40°C, en ce que l'étape b) est effectuée pendant un temps inférieur ou égal à 150 minutes et en ce que l'étape c) est effectuée pendant un temps compris entre 15 minutes et 18 heures.

11. Procédé selon la revendication 9 ou la revendication 10, **caractérisé en ce que** les étapes successives de préparation d'une solution d'au moins un dérivé fonctionnalisé de dextrane et éventuellement d'au moins un dextrane, d'imprégnation du support à l'aide de cette solution, de séchage du support, d'imprégnation du support à l'aide d'une solution, dans au moins un solvant organique, d'un agent de réticulation, et de réticulation dudit dérivé fonctionnalisé et éventuellement dudit dextrane sont, après séchage du support, réitérées au moins une fois, de préférence au moins deux à trois fois.

12. Procédé selon l'une quelconque des revendications 6 à 11, **caractérisé en ce que** l'étape c) de réticulation dudit dérivé fonctionnalisé de dextrane et éventuellement dudit dextrane est suivie d'une étape de lavage de la prothèse.

13. Procédé selon l'une quelconque des revendications 6 à 12, **caractérisé en ce que** ladite solution préparée lors de l'étape a) comprend, en outre, au moins un polysaccharide naturel ou synthétique fonctionnalisé au moins par des fonctions carboxylates et/ou sulfates.

14. Procédé selon l'une quelconque des revendications 6 à 13, **caractérisé en ce que** ladite solution préparée lors de l'étape a) comprend, en outre, au moins un additif choisi parmi les agents plastifiants et les agents assouplissants et/ou un ou plusieurs principes actifs sélectionnés dans le groupe constitué par les agents anticoagulants, les agents anti-bactériens et les agents anti-infectieux.

15. Prothèse vasculaire souples selon l'une quelconque des revendications 1 à,5, **caractérisée en ce qu'**elle est susceptible d'être obtenue par le procédé tel que défini dans l'une quelconque des revendications 6 à 14.

## Claims

1. Flexible vascular prosthesis, **characterised in that** it comprises a synthetic support impregnated with at least one covalently cross-linked, functionalised dextran derivative and optionally at least one covalently cross-linked dextran.

2. Prosthesis as claimed in claim 1, **characterised in that** the ratio by weight between the functionalised dextran derivative and dextran is between 1/99 and 30/70, preferably between 3/97 and 7/93, and even more preferably is equal to approximately 5/95.

3. Prosthesis as claimed in claim 1 or claim 2, **characterised in that** said synthetic support is additionally impregnated with at least one covalently cross-linked synthetic or natural polysaccharide functionalised by at least carboxylate and/or sulphate functions.

4. Prosthesis as claimed in claim 3, **characterised in that** the ratio by weight between the functionalised polysaccharide and dextran is advantageously between 1/99 and 30/70, preferably between 1/99 and 20/80.

5. Prosthesis as claimed in claim 3 or claim 4, **characterised in that** the ratio by weight between the functionalised dextran derivative and functionalised polysaccharide is advantageously between 1/99 and 99/1.

6. Method of preparing a vascular prosthesis as claimed in any one of the preceding claims, **characterised in that** it comprises the following steps:
a) preparing a solution of at least one functionalised dextran derivative and optionally at least one dextran,
b) impregnating the synthetic support with the aid of this solution,
c) cross-linking said functionalised dextran derivative and optionally said dextran.

7. Method as claimed in claim 6, **characterised in that**, between steps a) and b), it includes adding a cross-linking agent to the solution prepared during step a).

8. Method as claimed in claim 7, **characterised in that** steps b) and c) are carried out simultaneously at a pH of between 3 and 10, at a temperature of between 10 and 40°C and for a time of less than or equal to 150 minutes.

9. Method as claimed in claim 6, **characterised in that**, between steps b) and c), it includes a step of drying the support, followed by a step of impregnating the support with the aid of a solution of a cross-linking agent in at least one organic solvent.

10. Method as claimed in claim 9, **characterised in that** steps b) and c) are each carried out at a pH of between 3 and 10 and at a temperature of between 10 and 40°C, step b) is carried out for a time of less than or equal to 150 minutes and step c) is carried out for a time of between 15 minutes and 18 hours.

11. Method as claimed in claim 9 or claim 10, **characterised in that** the successive steps of preparing a solution of at least one functionalised dextran derivative and optionally at least one dextran, impregnating the support with the aid of this solution, drying the support, impregnating the support with the aid of a solution of a cross-linking agent in at least one organic solvent, and cross-linking said functionalised derivative and optionally said dextran are repeated at least once, preferably at least two to three times, after drying the support.

12. Method as claimed in any one of claims 6 to 11, **characterised in that** step c) involving the cross-linking of said functionalised dextran derivative and optionally said dextran is followed by a step of washing the prosthesis.

13. Method as claimed in any one of claims 6 to 12, **characterised in that** said solution prepared during step a) further comprises at least one natural or synthetic polysaccharide functionalised by at least carboxylate and/or sulphate functions.

14. Method as claimed in any one of claims 6 to 13, **characterised in that** said solution prepared during step a) further comprises at least one additive selected from plasticizing agents and agents to impart flexibility and/or one or more active ingredients selected from the group comprising anti-coagulants, anti-bacterial agents and anti-infection agents.

15. Flexible vascular prosthesis as claimed in any one of claims 1 to 5, **characterised in that** it is of the type which can be obtained by the method as defined in any one of claims 6 to 14.

## Patentansprüche

1. Geschmeidige Gefäßprothese, **dadurch gekennzeichnet, dass** sie einen synthetischen Träger umfasst, der mit wenigstens einem funktionalisierten, kovalent vernetzten Dextranderivat und gegebenenfalls wenigstens einem kovalent vernetzten Dextran imprägniert ist.

2. Prothese nach Anspruch 1, **dadurch gekennzeichnet, dass** das Gewichtsverhältnis zwischen dem funktionalisierten Dextranderivat und dem Dextran zwischen 1/99 und 30/70, bevorzugt zwischen 3/97 und 7/93, und besonders bevorzugt ungefähr gleich 5/95 ist.

3. Prothese nach Anspruch 1 oder Anspruch 2, **dadurch gekennzeichnet, dass** der synthetische Träger außerdem mit wenigstens einem kovalent vernetzten natürlichen oder synthetischen Polysaccharid imprägniert ist, das wenigstens mit Carboxylat- und/oder Sulfatfunktionen funktionalisiert ist.

4. Prothese nach Anspruch 3, **dadurch gekennzeichnet, dass** das Gewichtsverhältnis zwischen dem funktionalisierten Polysaccharid und dem Dextran vorteilhaft zwischen 1/99 und 30/70, bevorzugt zwischen 1/99 und 20/80, ist.

5. Prothese nach Anspruch 3 oder Anspruch 4, **dadurch gekennzeichnet, dass** das Gewichtsverhältnis zwischen dem funktionalisierten Dextranderivat und dem funktionalisierten Polysaccharid vorteilhaft zwischen 1/99 und 99/1 ist.

6. Verfahren zur Herstellung einer Gefäßprothese nach einem der vorhergehenden Ansprüche, **dadurch gekennzeichnet, dass** es die folgenden Schritte umfasst:
a) Herstellung einer Lösung von wenigstens einem funktionalisierten Dextranderivat und gegebenenfalls wenigstens einem Dextran,
b) Imprägnierung des synthetischen Trägers mit dieser Lösung,
c) Vernetzung des funktionalisierten Dextranderivats und gegebenenfalls des Dextrans.

7. Verfahren nach Anspruch 6, **dadurch gekennzeichnet, dass** es zwischen den Schritten a) und b) die Zugabe eines Vernetzungsmittels zu der in Schritt a) hergestellten Lösung umfasst.

8. Verfahren nach Anspruch 7, **dadurch gekennzeichnet, dass** die Schritte b) und c) bei einem pH-Wert zwischen 3 und 10, einer Temperatur zwischen 10 und 40°C und über einen Zeitraum von weniger oder gleich 150 Minuten gleichzeitig durchgeführt werden.

9. Verfahren nach Anspruch 6, **dadurch gekennzeichnet, dass** es zwischen den Schritten b) und c) einen Schritt zur Trocknung des Trägers, gefolgt von einem Schritt zur Imprägnierung des Trägers mit einer Lösung eines Vernetzungsmittels in wenigstens einem organischen Lösungsmittel umfasst.

10. Verfahren nach Anspruch 9, **dadurch gekennzeichnet, dass** die Schritte b) und c) jeweils bei einem pH-Wert zwischen 3 und 10 und einer Temperatur zwischen 10 und 40°C durchgeführt werden, dass Schritt b) über einen Zeitraum von weniger oder gleich 150 Minuten durchgeführt wird, und dass Schritt c) über einen Zeitraum zwischen 15 Minuten und 18 Stunden durchgeführt wird.

11. Verfahren nach Anspruch 9 oder Anspruch 10, **dadurch gekennzeichnet, dass** die aufeinanderfolgenden Schritte der Herstellung einer Lösung von wenigstens einem funktionalisierten Dextranderivat und gegebenenfalls wenigstens einem Dextran, der Imprägnierung des Trägers mit dieser Lösung, der Trocknung des Trägers, der Imprägnierung des Trägers mit einer Lösung eines Vernetzungsmittels in wenigstens einem organischen Lösungsmittel und der Vernetzung des funktionalisierten Derivats und gegebenenfalls des Dextrans nach Trocknung des Trägers wenigstens einmal, bevorzugt wenigstens zwei- bis dreimal, wiederholt werden.

12. Verfahren nach einem der Ansprüche 6 bis 11, **dadurch gekennzeichnet, dass** auf den Schritt c) der Vernetzung des funktionalisierten Dextranderivats und gegebenenfalls des Dextrans ein Schritt des Waschens der Prothese folgt.

13. Verfahren nach einem der Ansprüche 6 bis 12, **dadurch gekennzeichnet, dass** die in Schritt a) hergestellte Lösung außerdem wenigstens ein natürliches oder synthetisches Polysaccharid umfasst, das wenigstens mit Carboxylat- und/oder Sulfatfunktionen funktionalisiert ist.

14. Verfahren nach einem der Ansprüche 6 bis 13, **dadurch gekennzeichnet, dass** die in Schritt a) hergestellte Lösung außerdem wenigstens ein Hilfsmittel ausgewählt aus Plastifikatoren und Weichmachern und/oder einen oder mehrere Wirkstoffe ausgewählt aus der Gruppe bestehend aus Antikoagulantien, antibakteriellen Mitteln und infektionshemmenden Mitteln umfasst.

15. Geschmeidige Gefäßprothese nach einem der Ansprüche 1 bis 5, **dadurch gekennzeichnet, dass** sie nach dem Verfahren gemäß einem der Ansprüche 6 bis 14 erhältlich ist.
